# EUROPEAN PATENT APPLICATION

(11) **EP 3 967 997 A1**
(43) Date of publication of application: **16.03.2022**
(21) Application number: 20823487.2
(22) Date of filing: 05.06.2020
(51) Int. Cl.: G01L 7/00, G01L 27/00, A61M 1/36

(54) **PRESSURE SENSING DEVICE AND BLOOD PURIFICATION APPARATUS USING SAME**

(30) Priority: 12.06.2019 JP 2019109371
(71) Applicant: Nikkiso Co., Ltd., Tokyo 150-6022 (JP)
(72) Inventor: ISHIZAKI, Fumihiko, Tokyo 150-6022 (JP); MENJOH, Yuya, Makinohara-shi, Shizuoka 421-0496 (JP)
(74) Representative: von Hirschhausen, Helge
(86) International application number: PCT/JP2020/022406
(87) International publication number: WO 2020/250839

(57) **Abstract**

This pressure sensing device 1 is equipped with: a case 2 which is provided to a liquid passage 4 through which a liquid subject to pressure measurement is circulated; a diaphragm 3 that is provided so as to separate the space inside the case 2 into a first space 2a having the liquid circulated therethrough and a second space 2b having gas filled therein and that can be displaced in accordance with the pressure of the liquid within the first space 2a; a pressure sensor 6 for measuring the pressure of the gas filled in the second space 2b; and a diaphragm initial position adjusting mechanism 5 which is capable of adjusting the initial position of the diaphragm 3 to a desired position by adjusting the filling amount of the gas filled in the second space 2b, wherein the diaphragm initial position adjusting mechanism 5 has a reciprocating pump 51 that adjusts the filling amount of the gas filled in the second space 2b by a plunger driving part 513 causing a plunger 512 to reciprocate within a cylinder 511.

## Description

### Technical Field

The present invention relates to a pressure sensing device and a blood purification apparatus using the same.

### Background Art

In blood purification apparatuses, pressure sensing devices are used to measure liquid pressure of, e.g., blood or dialysate, etc. It is not preferable that blood and dialysate, etc., whose pressure is detected in the blood purification apparatuses, come into contact with air. For this reason, a pressure sensing device, which is configured to use a case (a pressure detection container) with a diaphragm provided so as to partition between a first space, through which a liquid whose pressure is to be detected, such as blood or dialysate, flows and a second space through which the liquid does not flow, and to measure pressure of a gas (air) filling the second space by a pressure sensor, is used (see, e.g., Patent Literature 1).

### Citation List

### Patent Literature

Patent Literature 1: Japanese Translation of PCT International Application Publication No. JP-T 2017/504389

### Summary of Invention

### Technical Problem

In case of the pressure sensing device described above, a detection value or pressure detection range of the pressure sensor changes depending on an initial position of the diaphragm. Thus, to accurately detect pressure and to make the pressure detection range a desired range, the initial position of the diaphragm is desired to be accurately adjusted to a given position.

Therefore, it is an object of the invention to provide a pressure sensing device capable of accurately adjusting an initial position of a diaphragm to a given position, and a blood purification apparatus using the same.

### Solution to Problem

To solve the problem mentioned above, the invention provides a pressure sensing device, comprising:
a case provided on a liquid flow path through which a liquid subject to pressure measurement flows;
a diaphragm that is provided so as to divide a space in the case into a first space through which the liquid flows and a second space through which the liquid does not flow, and can be displaced according to pressure of the liquid in the first space;
a pressure sensor to measure pressure of a gas filling the second space; and
a diaphragm initial position adjustment mechanism capable of adjusting an initial position of the diaphragm to a desired position by adjusting a filling amount of the gas filling the second space,
wherein the diaphragm initial position adjustment mechanism comprises a reciprocating pump that comprises a cylinder in communication with the second space, a plunger provided so as to be able to advance and retract within the cylinder, and a plunger driving part to advance and retract the plunger, and adjusts the filling amount of the gas filling the second space by advancing and retreating the plunger in the cylinder by the plunger driving part.

To solve the problem mentioned above, the invention also provides a blood purification apparatus, comprising:
the pressure sensing device described above as at least one of pressure sensing devices provided on a blood circuit to extracorporeally circulate blood of a patient, a liquid supply flow path to supply a supply liquid to the blood circuit or to a blood purifier provided on the blood circuit, and a waste liquid flow path to discharge a waste liquid from the blood purifier.

### Advantageous Effects of Invention

According to the invention, it is possible to provide a pressure sensing device capable of accurately adjusting an initial position of a diaphragm to a given position, and a blood purification apparatus using the same.

### Brief Description of Drawings

FIG. 1 is a schematic configuration diagram illustrating a blood purification apparatus in an embodiment of the present invention.
FIG. 2A is a schematic configuration diagram illustrating a pressure sensing device in the embodiment of the invention.
FIG. 2B is a cross-sectional view showing a case.
FIG. 2C is a schematic configuration diagram illustrating a reciprocating pump.
FIG. 3 is a flowchart showing a control flow executed by a control unit.
FIG. 4A is a flowchart showing a modification of the control flow executed by the control unit.
FIG. 4B is a flowchart showing a modification of the control flow executed by the control unit.
FIG. 5 is a flowchart showing a modification of the control flow executed by the control unit.
FIG. 6A is a schematic configuration diagram illustrating the pressure sensing device in another embodiment of the invention.
FIG. 6B is a flowchart showing the control flow executed by the control unit of the pressure sensing device of FIG. 6A.
FIG. 7A is a schematic configuration diagram illustrating the pressure sensing device in another embodiment of the invention.
FIG. 7B is a flowchart showing the control flow executed by the control unit of the pressure sensing device of FIG. 7A.

### Description of Embodiments

### (Embodiment)

An embodiment of the invention will be described below in conjunction with the appended drawings.

### (Blood purification apparatus)

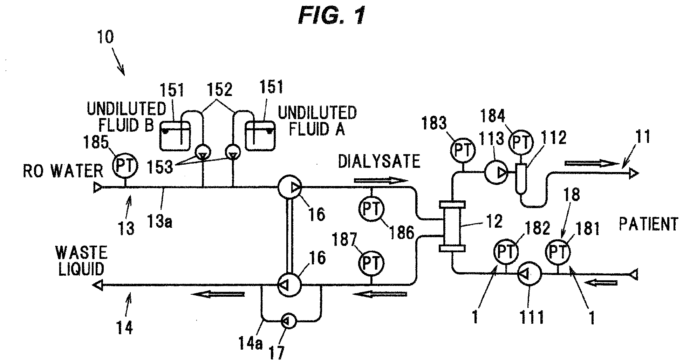

Firstly, a blood purification apparatus in which a pressure sensing device of the present embodiment is used will be described. FIG. 1 is a schematic configuration diagram illustrating a blood purification apparatus in the present embodiment.

As shown in FIG. 1, a blood purification apparatus 10 includes a liquid supply flow path 13 to supply a supply liquid to a blood circuit 11 extracorporeally circulating blood of a patient or to a blood purifier 12 provided on the blood circuit 11, and a waste liquid flow path 14 to discharge a waste liquid from the blood purifier 12. FIG. 1 shows an example in which the liquid supply flow path 13 is a dialysate flow path 13a to supply a dialysate to the blood purifier 12. However, it is not limited thereto, and the liquid supply flow path 13 may be a replenishing liquid flow path to supply a replenishing liquid directly to the blood circuit 11, or may include both the dialysate flow path 13a and the replenishing liquid flow path.

The blood circuit 11 is composed of, e.g., a flexible tube, etc. A blood pump 111, the blood purifier 12, a second blood pump 113 and an air trap chamber 112 are sequentially provided on the blood circuit 11 from the upstream to the downstream of a blood flow. The blood pump 111 is a liquid feed pump to send blood. The air trap chamber 112 is a device to remove air bubbles from the blood.

A first pressure sensing device 181 to measure pressure of blood on the upstream side of the blood pump 111 is provided on the upstream side of the blood pump 111 (at upstream of the blood flow). A second pressure sensing device 182 to measure pressure of the blood on the upstream side of the blood purifier is provided between the blood pump 111 and the blood purifier 12. A third pressure sensing device 183 to measure pressure of the blood on the downstream side of the blood purifier is provided between the blood purifier 12 and the second blood pump 113. A fourth pressure sensing device 184 to measure pressure inside the air trap chamber 112 is provided on the air trap chamber 112. The third and fourth pressure sensing devices 183 and 184 are essential when the second blood pump 113 is provided, but the third pressure sensing device 183 can be omitted when the second blood pump 113 is omitted.

From a RO (Reverse Osmosis) device (not shown) which produces clean dialysis water using a reverse osmosis membrane (RO membrane), dialysis water is supplied to the dialysate flow path 13a. Two types of undiluted dialysate fluids, an undiluted fluid A and an undiluted fluid B, are also supplied to the dialysate flow path 13a. The two undiluted fluids are respectively stored in undiluted fluid storage tanks 151, and the undiluted fluid A and the undiluted fluid B are supplied to the dialysate flow path 13a respectively from the undiluted fluid storage tanks 151 via undiluted fluid flow paths 152. Undiluted fluid injection pumps 153, which are liquid feed pumps pumping out the undiluted fluid A or the undiluted fluid B, are respectively provided on the two undiluted fluid flow paths 152. The undiluted fluid A and the undiluted fluid B are mixed with the dialysis water in the dialysate flow path 13a and the dialysate is thereby prepared. The prepared dialysate is introduced into the blood purifier 12 via a duplex pump 16.

A fifth pressure sensing device 185 to measure pressure of supplying the dialysis water is provided on the dialysate flow path 13a on the upstream side relative to portions connected to the undiluted fluid flow paths 152. A sixth pressure sensing device 186 to measure pressure of the dialysate to be introduced into the blood purifier 12 is provided on the dialysate flow path 13a between the duplex pump 16 and the blood purifier 12. In addition, a seventh pressure sensing device 187 to measure pressure of the waste liquid discharged from the blood purifier 12 is provided on the waste liquid flow path 14 between the blood purifier 12 and the duplex pump 16.

The waste liquid from the blood purifier 12 is discharged through the waste liquid flow path 14. The duplex pump 16 is provided over the dialysate flow path 13a and the waste liquid flow path 14 and performs pump operation so that an amount of the dialysate introduced into the blood purifier 12 is equal to an amount of the waste liquid discharged from the blood purifier 12. In addition, a water removal flow path 14a is provided on the waste liquid flow path 14 so as to bypass the dual pump 16, and a water removal pump 17 is provided on the water removal flow path 14a. When the water removal pump 17 is driven, the amount of the waste liquid discharged from the blood purifier 12 becomes larger than the amount of the dialysate introduced into the blood purifier 12 and water is removed from the blood. It is possible to adjust the amount of water removed from the blood by adjusting the amount of liquid sent by the water removal pump 17.

In the blood purification apparatus 10 of the present embodiment, at least one of the pressure sensing devices 181 - 187 provided on the blood circuit 11, the liquid supply flow path 13 (the dialysate flow path 13a in this example) and the waste liquid flow path 14 is a pressure sensing device 1 in the present embodiment. In the blood purification apparatus 10, the pressure sensing device 1 in the present embodiment may be used as at least one of the first to seventh pressure sensing devices 181 - 187, or the pressure sensing device 1 in the present embodiment may be used as a pressure sensing device provided at another position.

The configuration of FIG. 1 is only an example and a specific configuration of the blood purification apparatus 10 can be changed appropriately.

### (Pressure sensing device 1)

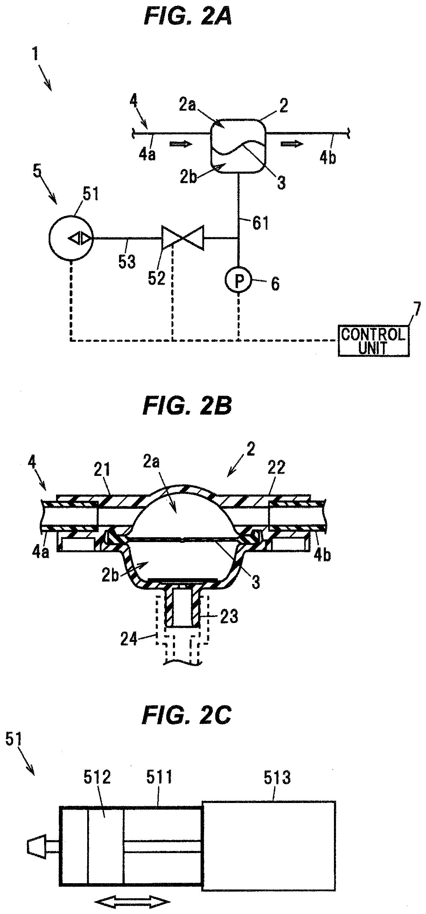

FIG. 2A is a schematic configuration diagram illustrating the pressure sensing device 1 in the present embodiment, FIG. 2B is a cross-sectional view showing a case, and FIG. 2C is a schematic configuration diagram illustrating a reciprocating pump.

As shown in FIGS. 2A to 2C, the pressure sensing device 1 includes a case 2 provided on a liquid flow path 4 (the blood circuit 11 in this example) through which a liquid subject to pressure measurement (blood in this example) flows, a diaphragm 3 dividing a space in the case 2 into a first space 2a with the liquid (blood) flowing therethrough and a second space 2b filled with a gas (air in this example), a pressure sensor 6 to measure pressure of the gas filling the second space 2b, a diaphragm initial position adjustment mechanism 5 capable of adjusting an initial position of the diaphragm 3 to a desired position by adjusting a filling amount of the gas filling the second space 2b, and a control unit 7 that controls the diaphragm initial position adjustment mechanism 5. Although the liquid subject to pressure measurement is blood and the gas filling the second space 2b is air in the following description, the liquid subject to measurement and the gas filling the second space 2b are not limited thereto. In addition, in the following description, the term simply referred as "air" means the air filling the second space 2b, etc.

The liquid flow path 4 has an inflow path 4a to let the blood flow into the first space 2a and an outflow path 4b to let the blood flow out of the first space 2a. When the pressure sensing device 1 is used as, e.g., the second pressure sensing device 182 in FIG. 1, the inflow path 4a serves as the blood circuit 11 extending from the blood pump 111 and the outflow path 4b serves as the blood circuit 11 extending to the blood purifier 12.

The case 2 is composed of a hard resin molded article, etc., and is provided on the liquid flow path 4. The case 2 integrally includes a first connection part 21 being in communication with the first space 2a and connected to the inflow path 4a and a second connection part 22 being in communication with the first space 2a and connected to the outflow path 4b. The case 2 also integrally includes a protruding part 23 that is in communication with the second space 2b and protrudes outward. The pressure sensing device 1 also includes a socket part 24 which is provided separately from the case 2 and into which the protruding part 23 is inserted and connected. Although it is not shown, a measurement flow path 61 (described later) is connected to the socket part 24, and the measurement flow path 61 is communicated with the second space 2b by inserting and connecting the protruding part 23 to the socket part 24. The case 2 is configured to be removable from the socket part 24 by detaching the protruding part 23 from the socket part 24, which allows the case 2 to be disposable. Thus, adjustment of the initial position of the diaphragm 3 (described later) is performed every time a new case 2 is attached. In this regard, the case 2 does not need to be entirely disposable and may be configured to be splittable on, e.g., the second space 2b side relative to the diaphragm 3 so that only a portion of the case 2 including the first space 2a can be disposable. It is not necessary to separately form the case 2 and the socket part 24, and the case 2 may be integrally formed with the socket part 24.

The diaphragm 3 is a flexible membrane and is provided in the case 2 so as to divide an internal space of the case 2 into two spaces, the first space 2a and the second space 2b. Materials of the case 2 and the diaphragm 3 are not specifically limited. The diaphragm 3 is configured to be displaceable according to pressure of the blood in the first space 2a and serves to transmit the pressure of the blood in the first space 2a to the air in the second space 2b.

The pressure sensor 6 is connected to the second space 2b of the case 2 via the measurement flow path 61 and measures pressure of the air filling the second space 2b and the measurement flow path 61. A detection signal of the pressure sensor 6 is output to the control unit 7.

The diaphragm initial position adjustment mechanism 5 has a reciprocating pump 51, a pump flow path 53 connecting the reciprocating pump 51 to the second space 2b, and a pump flow path opening/closing valve 52 that is provided on the pump flow path 53 and serves as a pump flow path opening/closing mechanism to open/close the pump flow path 53. Although the pump flow path 53 in the present embodiment is connected to the second space 2b via the measurement flow path 61, the pump flow path 53 may be directly connected to the second space 2b of the case 2. As the pump flow path opening/closing valve 52, it is desirable to use a solenoid valve so that opening/closing can be controlled automatically, but, e.g., a clamp mechanism, etc., may be used. In the present embodiment, a solenoid valve is used as the pump flow path opening/closing valve 52.

The reciprocating pump 51 is also called a plunger pump or a piston pump, and has a cylinder 511 in communication with the second space 2b via the pump flow path 53 and the measurement flow path 61, a plunger 512 (or a piston) provided so as to be able to advance and retract within the cylinder 511, and a plunger driving part 513 to advance and retract the plunger 512. The cylinder 511 and the pump flow path 53 are filled with the air in the same manner as the second space 2b and the measurement flow path 61, and by advancing and retracting the plunger 512 in the cylinder 511 by the plunger driving part 513, it is possible to adjust the filling amount of the air filling the second space 2b and thereby adjust the position of the diaphragm 3. As the plunger driving part 513, it is possible to use, e.g., a stepping motor.

The control unit 7 adjusts the initial position of the diaphragm 3 by controlling the reciprocating pump 51 and the pump flow path opening/closing valve 52. The control unit 7 is realized by appropriately combining an arithmetic element such as CPU, a storage device such as memory, a software, and an interface, etc.

In the present embodiment, the filling amount of the air filling the second space 2b, the measurement flow path 61, the cylinder 511 and the pump flow path 53 is known. In this case, since it is known where to position the plunger 512 to cause the diaphragm 3 to be located at a desired position, the control unit 7 controls to move the pusher 512 to a predetermined position (referred to as an adjusted position) so that the diaphragm 3 is located at a desired position.

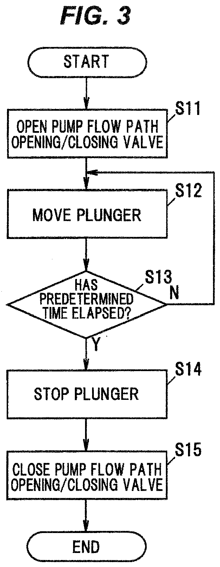

In particular, as shown in FIG. 3, the control unit 7 firstly opens the pump flow path opening/closing valve 52 in Step S11, moves the plunger 512 from its initial position (e.g., retracts toward the decompression side from a position of the plunger 512 when fully pushed, or advances toward the compression side from a position of the plunger 512 when fully withdrawn) in Step S12, and determines whether a predetermined time has elapsed in Step S13. Alternatively, whether a travel amount of the plunger 512 has reached a predetermined travel amount may be determined in Step S13. When the determination made in Step S13 is NO, the process returns to Step S12. When the determination made in Step S13 is YES, the plunger 512 is stopped in Step S14, the pump flow path opening/closing valve 52 is then closed in Step S15 and the process ends. The time taken for the determination in Step S13 (the predetermined time mentioned above) is set to the time required to move the plunger 512 from the initial position to the adjusted position. It is desirable that the control unit 7 adjust the initial position of the diaphragm 3 by executing the control flow of FIG. 3 prior to blood purification treatment, e.g., during priming.

In the present embodiment, to adjust the filling amount of the air in the second space 2b by moving the plunger 512, it is possible to linearly change pressure of the air. When, e.g., a peristaltic pump is used as a means to compress and decompress the air, the pressure of the air changes stepwise and it is thus difficult to precisely adjust the initial position of the diaphragm 3. That is, by using the reciprocating pump 51 as in the present embodiment, it is possible to adjust the initial position of the diaphragm 3 more precisely. In addition, the peristaltic pump is configured to squeeze a flexible tube and variation in the discharge amount occurs due to production tolerance on the tube or deterioration of the tube over time, but the reciprocating pump 51 does not use any tube and such a problem can be suppressed. In addition, the tube of the peristaltic pump needs to be periodically replaced, but the reciprocating pump 51 does not need such periodic replacement of component and thus has high durability, and also contributes to cost reduction by reducing the number of components.

Furthermore, by configuring such that the pump flow path 53 can be closed by the pump flow path opening/closing valve 52 after adjusting the initial position of the diaphragm 3 by the reciprocating pump 51, it is possible to reduce the filling amount of the air whose pressure is transmitted from the second space 2b to the pressure sensor 6. Since the size of the pressure sensing device 1 is determined by the filling amount of the air whose pressure is transmitted from the second space 2b to the pressure sensor 6, the size of the pressure sensing device 1 can be further reduced by providing the pump flow path opening/closing valve 52. However, the pump flow path opening/closing valve 52 is not essential and can be omitted.

If the diaphragm 3 is damaged, the air leaks into the blood circuit 11 when performing compression by the reciprocating pump 51 and thus cannot be compressed. Thus, it is also possible to detect the failure of the pressure sensing device 1 based on an output value of the pressure sensor 6 when compression is performed by the reciprocating pump 51.

### (Modifications)

The filling amount of the air filling the second space 2b, the measurement flow path 61, the cylinder 511 and the pump flow path 53 is known in the present embodiment, but a means to estimate the filling amount of the air is required when the filling amount of the air is unknown. The filling amount of the air can be estimated from, e.g., a change in pressure of the air when the plunger 512 is moved. In this case, the control unit 7 estimates the filling amount of the air based on a travelled distance of the plunger 512 and a change in pressure of the air at that time, calculates the adjusted position, which is a position of the plunger 512 causing the diaphragm 3 to be located at a desired position, based on the estimated filling amount of the air, and then moves the plunger 512 to the adjusted position. When estimating the filling amount of the air, it may be estimated using, e.g., Boyle-Charles's law.

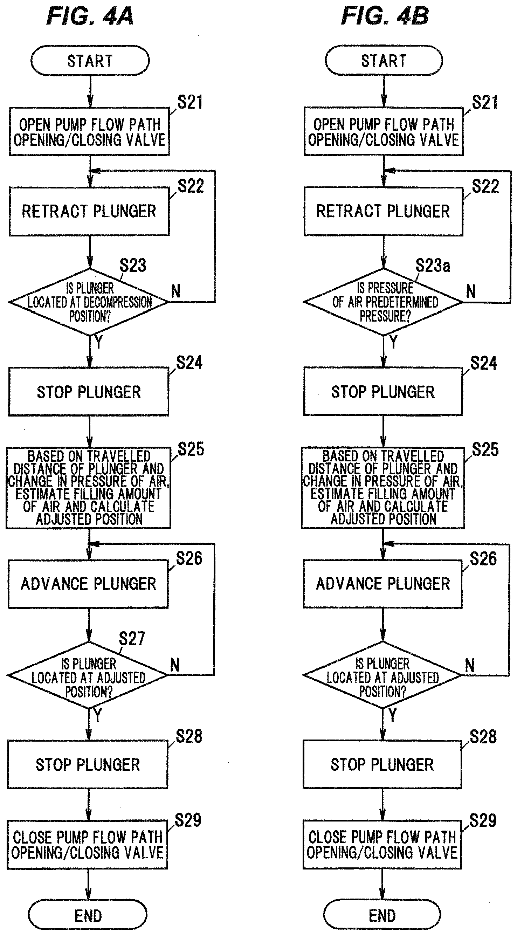

FIG. 4A shows a control flow when estimating the filling amount of the air based on the travelled distance of the plunger 512. As shown in FIG. 4A, the control unit 7 firstly opens the pump flow path opening/closing valve 52 in Step S21, retracts the plunger 512 from the initial position (e.g., retracts toward the decompression side from a position of the plunger 512 when fully pushed) in Step S22, and determines whether the plunger 512 has reached a predetermined decompression position (whether the plunger 512 has been moved by a predetermined distance) in Step S23. When the determination made in Step S23 is NO, the process returns to Step S22. When the determination made in Step S23 is YES, the plunger 512 is stopped in Step S24, and then, the filling amount of the air is estimated based on the travelled distance of the plunger 512 and a pressure change of the air detected by the pressure sensor 6 and the adjusted position of the plunger 512 causing the diaphragm 3 to be located at a desired position is calculated in Step S25. After that, the plunger 512 is advanced in Step S26, and whether the plunger 512 has reached the adjusted position is determined in Step S27. When the determination made in Step S27 is NO, the process returns to Step S26. When the determination made in Step S27 is YES, the plunger 512 is stopped in Step S28, the pump flow path opening/closing valve 52 is then closed in Step S29 and the process ends.

FIG. 4B shows a control flow when executed based on the pressure change of the air. The control flow of FIG. 4B is a modification of the control flow of FIG. 4A in which Step S23 is replaced with Step S23a of determining whether the pressure of the air is a predetermined pressure. That is, the pressure change of the air when moving the plunger 512 by a predetermined distance is detected in FIG. 4A, while the travelled distance of the plunger 512 causing the pressure change of the air to be a predetermined value is detected in FIG. 4B. With either of these methods, it is possible to estimate the filling amount of the air and calculate the adjusted position of the plunger 512.

In the examples of FIGS. 4A and 4B, a plunger position detection unit to detect the position of the plunger 512 is required. As the plunger position detection unit, it is possible to use, e.g., an encoder that detects a rotational speed of a motor used for the plunger driving part 513, etc., or a linear potentiometer that directly detects the position of the plunger 512, etc. In addition, when using a stepping motor (pulse motor) as the plunger driving part 513, it is possible to detect the position of the plunger 512 also based on a driving amount (a number of output pulses) of the stepping motor. When operating the plunger 512 at a constant speed, it is also possible to detect the plunger 512 from the operating time. Furthermore, as the plunger position detection unit, it also possible to use a contact type sensor such as limit switch, strain gauge or piezoelectric element sensor and it is also possible to use an indirect sensor such as photoelectric sensor or pressure sensor.

A diaphragm position detection unit capable of detecting that the diaphragm is located at a predetermined position (e.g., a position at which the volume of the first space 2a reaches the maximum or the minimum) may be further provided. The diaphragm position detection unit may be configured to detect the position of the diaphragm 3 using, e.g., a sensor or the like such as photoelectric sensor. It is also possible to estimate the position of the diaphragm 3 by using the detection result of the encoder or linear potentiometer mentioned above, or the driving amount of the stepping motor, or an output of the pressure sensor 6.

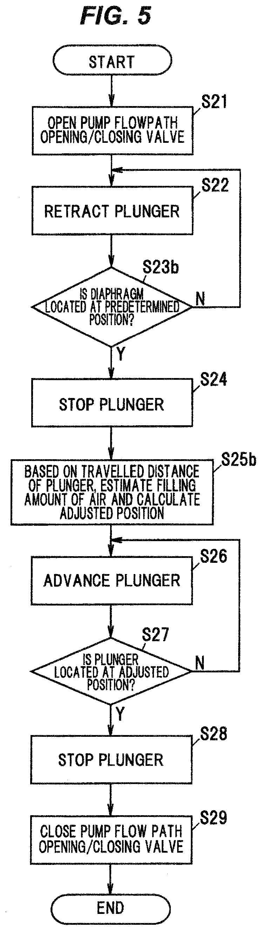

When the diaphragm position detection unit is provided, the control unit 7 preferably estimate the filling amount of the air based on the travel amount of the plunger 512 until the diaphragm position detection unit detects that the diaphragm 3 is located at a predetermined position since the plunger 512 is moved from the initial position. In more particular, the filling amount of the air can be estimated from a retraction distance of the plunger 512 which is required for the diaphragm 3 to reach the predetermined position (e.g., the position at which the volume of the first space 2a reaches the maximum) from the no-load position. The control flow in this case is shown in FIG. 5. The control flow of FIG. 5 is a modification of the control flow of FIG. 4A in which Step S23 is replaced with Step S23b of determining whether the diaphragm 3 is located at a predetermined position and Step S25 is replaced with Step S25b of estimating the filling amount of the air based on the travelled distance of the plunger 512 and calculating the adjusted position.

### (Functions and Effects of the embodiment)

As described above, the pressure sensing device 1 in the present embodiment includes the diaphragm initial position adjustment mechanism 5 capable of adjusting the initial position of the diaphragm 3 to a desired position by adjusting the filling amount of the air filling the second space 2b, and the diaphragm initial position adjustment mechanism 5 has the reciprocating pump 51 that adjusts the filling amount of the gas filling the second space 2b by advancing and retreating the plunger 512 in the cylinder 511.

By adjusting the initial position of the diaphragm 3 using the reciprocating pump 51, the initial position of the diaphragm 3 can be accurately adjusted to a given position and variation in the initial position of the diaphragm 3 can be suppressed, as compared to when a peristaltic pump, etc., is used. By suppressing the variation in the initial position of the diaphragm 3, it is possible to improve repeat accuracy of the initial position adjustment of the diaphragm 3 which is performed every time the case 2 is changed, it is possible to improve pressure detection accuracy and also possible to accurately adjust the pressure detection range. In addition, when the variation in the initial position of the diaphragm 3 is large, the filling amount of the air filling the second space 2b and the measurement flow path 61 needs to be increased to absorb the variation. However, in the present embodiment, since the variation in the initial position of the diaphragm 3 can be suppressed, the filling amount of the air can be reduced and this contributes to size reduction of the pressure sensing device 1.

### (Other embodiments)

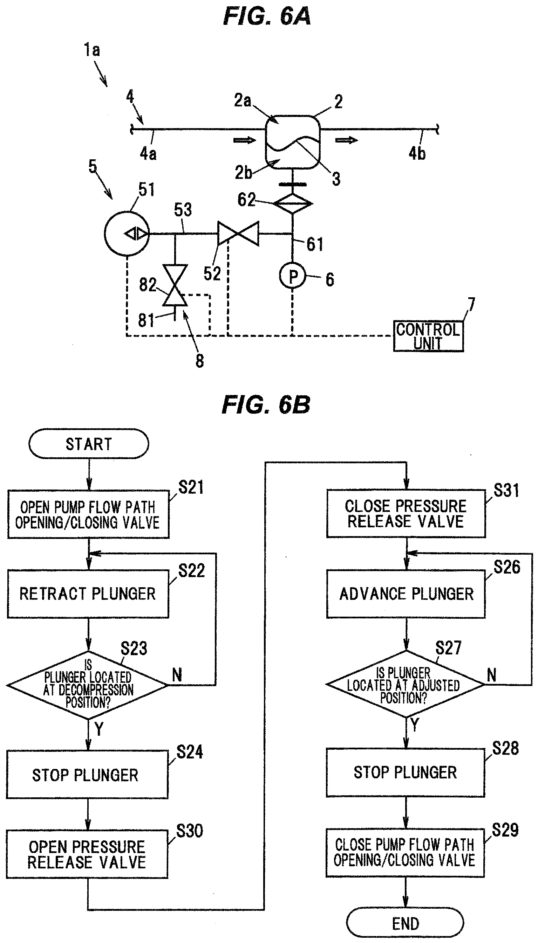

A pressure sensing device 1a shown in FIG. 6A is a device in which the pressure sensing device 1 of FIG. 2A includes a pressure release mechanism 8 capable of releasing the air filling the second space 2b, etc., to the atmosphere. The pressure release mechanism 8 has a pressure release flow path 81 with one end connected to the pump flow path 53 between the reciprocating pump 51 and the pump flow path opening/closing valve 52 and the other end opened to the atmosphere, and a pressure release valve 82 that is provided on the pressure release flow path 81 and is capable of opening/closing the pressure release flow path 81. Opening/closing of the pressure release valve 82 is controlled by the control unit 7. A solenoid valve is used as the pressure release valve 82 in the present embodiment, but, e.g., a clamp mechanism may be used.

The control unit 7 of the pressure sensing device 1a retracts the plunger 512 from the initial position and moves to a predetermined decompression position (a pressure application position), releases the air to the atmosphere by opening the pressure release valve 82 and then closes the pressure release valve 82, and after that, moves the plunger 512 to the adjusted position which is a position of the plunger 512 causing the diaphragm 3 to be located at a desired position. In this regard, after the plunger 512 is advanced from the initial position and moved to a predetermined compression position, it may be opened to the atmosphere.

When a new case 2 is attached to the socket part 24, the position of the diaphragm 3 in the case 2 in the unadjusted state can be various positions. In this case, by controlling to open to the atmosphere after moving the diaphragm 3 in one direction by the reciprocating pump 51, the diaphragm 3 can be positioned at a fixed position (the no-load position) and also the air filling the second space 2b, etc., can be brought to atmospheric pressure, thereby making the filling amount of the air a known amount. Thereafter, by moving the plunger 512 to a predetermined adjusted position, the diaphragm 3 can be accurately adjusted to a desired position.

FIG. 6B shows the control flow executed by the control unit 7 of the pressure sensing device 1a. The control flow of FIG. 6B is a modification of the control flow of FIG. 4A in which Step S25 is replaced with Step S30 of opening the pressure release valve 82 and Step S31 of closing the pressure release valve 82 after Step S30.

The pressure sensing device 1a also includes an air filter 62 that is provided in the measurement flow path 61 to prevent foreign matter from entering the piping. The air filter 62 is a so-called hydrophobic filter, and is configured to allow gases to pass therethrough but to not allow liquids to pass therethrough (very high resistance to the passage of liquids).

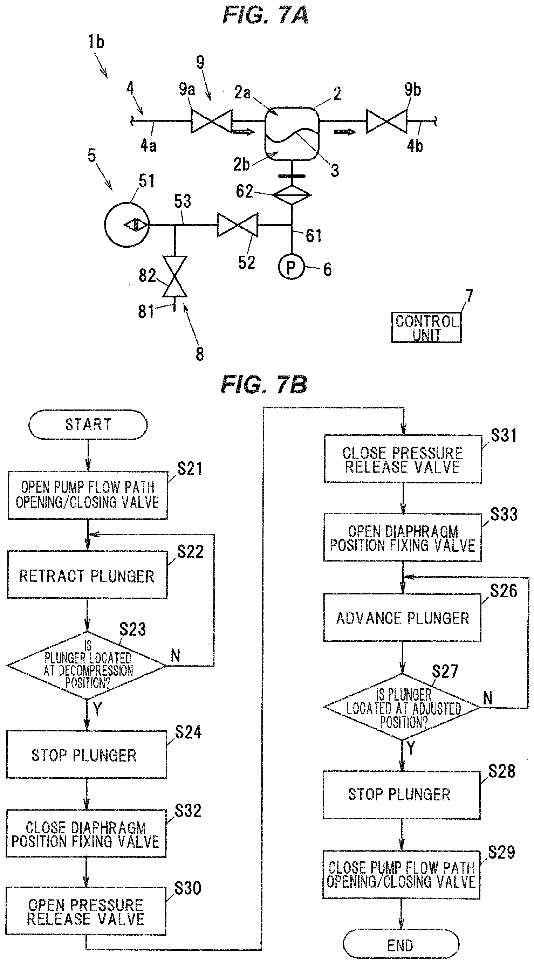

A pressure sensing device 1b shown in FIG. 7A is a device in which the pressure sensing device 1a of FIG. 6A further includes a diaphragm position fixing mechanism 9 capable of fixing the position of the diaphragm 3 by closing the liquid flow path 4. The diaphragm position fixing mechanism 9 is configured to be capable of simultaneously closing both the inflow path 4a and the outflow path 4b. In this embodiment, the diaphragm position fixing mechanism 9 has a first diaphragm position fixing valve 9a which is provided on the inflow path 4a and is capable of opening/closing the inflow path 4a, and a second diaphragm position fixing valve 9b which is provided on the outflow path 4b and is capable of opening/closing the outflow path 4b. By closing both the diaphragm position fixing valves 9a and 9b, the liquid flow path 4 between the two diaphragm position fixing valves 9a and 9b, including the first space 2a, is closed and the position of the diaphragm 3 is fixed. Although solenoid valves are used as both diaphragm position fixing valves 9a and 9b in this embodiment, e.g., a clamp mechanism may be used. Opening/closing of both diaphragm position fixing valves 9a and 9b is controlled by the control unit 7. In this embodiment, the pump flow path opening/closing valve 52 also serves as the diaphragm position fixing mechanism 9.

The control unit 7 of the pressure sensing device 1b opens the pressure release valve 82 after the position of the diaphragm 3 is fixed by the diaphragm position fixing mechanism 9. In particular, as shown in FIG. 7B, Step S32 of fixing the diaphragm 3 by closing both diaphragm position fixing valves 9a and 9b is inserted between Step S24 and Step S30 in the control flow of FIG. 6B, and Step S33 of releasing the diaphragm 3 from the fixed position by opening both diaphragm position fixing valves 9a and 9b is inserted between Step S31 and Step S26. In this regard, Step S33 (opening both diaphragm position fixing valves 9a and 9b) may be performed after returning the plunger 512 to the adjusted position, i.e., after Step S28.

As a result, the diaphragm 3 does not return to the no-load position when opening the pressure release valve 82 in Step S30 and is held at a position at which, e.g., the volume of the first space 2a is the largest. Therefore, as compared to FIG. 6A and FIG. 6B in which the diaphragm 3 returns to the no-load position, the position of the diaphragm 3 when opened to the atmosphere can be kept more fixed, and accuracy of position adjustment of the diaphragm 3 is further improved.

### (Summary of the embodiment)

Technical ideas understood from the embodiments will be described below citing the reference numerals, etc., used for the embodiments. However, each reference numeral, etc., described below is not intended to limit the constituent elements in the claims to the members, etc., specifically described in the embodiments.

[1] A pressure sensing device (1), comprising: a case (2) provided on a liquid flow path through which a liquid subject to pressure measurement flows; a diaphragm (3) that is provided so as to divide a space in the case (2) into a first space (2a) with the liquid flowing therethrough and a second space (2b) filled with a gas and can be displaced according to pressure of the liquid in the first space (2b); a pressure sensor (6) to measure pressure of the gas filling the second space (2b); and a diaphragm initial position adjustment mechanism (5) capable of adjusting an initial position of the diaphragm (3) to a desired position by adjusting a filling amount of the gas filling the second space (2b), wherein the diaphragm initial position adjustment mechanism (5) comprises a reciprocating pump (51) that comprises a cylinder (511) in communication with the second space (2b), a plunger (512) provided so as to be able to advance and retract within the cylinder (511), and a plunger driving part (53) to advance and retract the plunger (512), and adjusts a filling amount of the gas filling the second space (2b) by advancing and retreating the plunger (512) in the cylinder (511) by the plunger driving part (513).
[2] The pressure sensing device (1) described in [1], wherein the diaphragm initial position adjustment mechanism (5) comprises a pump flow path (52) connecting the reciprocating pump (51) to the second space (2b), and a pump flow path opening/closing mechanism (53) provided on the pump flow path (52) to open/close the pump flow path (52).
[3] The pressure sensing device (1) described in [1] or [2], comprising: a control unit (7) that adjusts the initial position of the diaphragm (3) by controlling the reciprocating pump (51), wherein the control unit (7) estimates the filling amount of the gas based on a travelled distance of the plunger (512) and a change in pressure of the gas at that time, calculates an adjusted position, which is a position of the plunger at which the diaphragm (3) is located at a desired position, based on the estimated filling amount of the gas, and moves the plunger (512) to the adjusted position.
[4] The pressure sensing device (1) described in [1] or [2], comprising: a control unit (7) that adjusts the initial position of the diaphragm (3) by controlling the reciprocating pump (51); and a diaphragm position detection unit capable of detecting that the diaphragm (3) is located at a predetermined position, wherein the control unit (7) estimates the filling amount of the gas based on a travel amount of the plunger (512) until the diaphragm position detection unit detects that the diaphragm (3) is located at the predetermined position since the plunger (512) is moved from the initial position, calculates an adjusted position, which is a position of the plunger (512) at which the diaphragm (3) is located at a desired position, based on the estimated filling amount of the gas, and moves the plunger (512) to the adjusted position.
[5] The pressure sensing device (la) described in [1] or [2], comprising: a pressure release mechanism (8) comprising a pressure release valve (82) capable of releasing the gas to the atmosphere; and a control unit (7) that adjusts the initial position of the diaphragm (3) by controlling the reciprocating pump (51) and the pressure release mechanism (8), wherein the control unit (7) advances or retracts the plunger (512) to a predetermined pressure application position, closes the pressure release valve (82) after opening the pressure release valve (82) and releasing the gas to the atmosphere, and then moves the plunger (512) to an adjusted position which is a position of the plunger (512) at which the diaphragm (3) is located at a desired position.
[6] The pressure sensing device (1b) described in [5], comprising: a diaphragm position fixing mechanism (9) capable of fixing the position of the diaphragm (3) by closing the liquid flow path (4), wherein the control unit (7) opens the pressure release valve (82) after the position of the diaphragm (3) is fixed by the diaphragm position fixing mechanism (9).
[7] The pressure sensing device (1) described in any one of [1] to [6], comprising: a socket part (24) to which the pressure sensor (6) is connected, wherein the case (2) and the diaphragm (3) are provided so as to be removable from the socket part (24).
[8] A blood purification apparatus (10), comprising: a blood circuit (11) to extracorporeally circulate blood of a patient; a liquid supply flow path (13) to supply a supply liquid to the blood circuit (11) or to a blood purifier (12) provided on the blood circuit (11); and a waste liquid flow path (14) to discharge a waste liquid from the blood purifier (12), wherein at least one of pressure sensing devices (181 - 187) provided on the blood circuit (11), the liquid supply flow path (13) and the waste liquid flow path (14) comprises the pressure sensing device (1, 1a, 1b) described any one of [1] to [7].

Although the embodiments of the invention have been described, the invention according to claims is not to be limited to the embodiments described above. In addition, not all combinations of the features described in the embodiments are necessary to solve the problem of the invention. In addition, the invention can be appropriately modified and implemented without departing from the gist thereof.

### Reference Signs List

- 1: pressure sensing device
- 2: case
- 2a: first space
- 2b: second space
- 3: diaphragm
- 4: liquid flow path
- 5: diaphragm initial position adjustment mechanism
- 51: reciprocating pump
- 511: cylinder
- 512: plunger
- 513: plunger driving part
- 52: pump flow path opening/closing valve
- 53: pump flow path
- 6: pressure sensor
- 7: control unit
- 8: pressure release mechanism
- 82: pressure release valve
- 9: diaphragm position fixing mechanism
- 10: blood purification apparatus
- 11: blood circuit
- 12: blood purifier
- 13: liquid supply flow path
- 14: waste liquid flow path

## Claims

1. A pressure sensing device, comprising:
a case provided on a liquid flow path through which a liquid subject to pressure measurement flows;
a diaphragm that is provided so as to divide a space in the case into a first space with the liquid flowing therethrough and a second space filled with a gas and can be displaced according to pressure of the liquid is the first space;
a pressure sensor to measure pressure of the gas filling the second space; and
a diaphragm initial position adjustment mechanism capable of adjusting an initial position of the diaphragm to a desired position by adjusting a filling amount of the gas filling the second space,
wherein the diaphragm initial position adjustment mechanism comprises a reciprocating pump that comprises a cylinder in communication with the second space, a plunger provided so as to be able to advance and retract within the cylinder, and a plunger driving part to advance and retract the plunger, and adjusts a filling amount of the gas filling the second space by advancing and retreating the plunger in the cylinder by the plunger driving part.

2. The pressure sensing device according to claim 1, wherein the diaphragm initial position adjustment mechanism comprises a pump flow path connecting the reciprocating pump to the second space, and a pump flow path opening/closing mechanism provided on the pump flow path to open/close the pump flow path.

3. The pressure sensing device according to claim 1 or 2, comprising:
a control unit that adjusts the initial position of the diaphragm by controlling the reciprocating pump,
wherein the control unit estimates the filling amount of the gas based on a travelled distance of the plunger and a change in pressure of the gas at that time, calculates an adjusted position, which is a position of the plunger at which the diaphragm is located at a desired position, based on the estimated filling amount of the gas, and moves the plunger to the adjusted position.

4. The pressure sensing device according to claim 1 or 2, comprising:
a control unit that adjusts the initial position of the diaphragm by controlling the reciprocating pump; and
a diaphragm position detection unit capable of detecting that the diaphragm is located at a predetermined position,
wherein the control unit estimates the filling amount of the gas based on a travel amount of the plunger until the diaphragm position detection unit detects that the diaphragm is located at the predetermined position since the plunger is moved from the initial position, calculates an adjusted position, which is a position of the plunger at which the diaphragm is located at a desired position, based on the estimated filling amount of the gas, and moves the plunger to the adjusted position.

5. The pressure sensing device according to claim 1 or 2, comprising:
a pressure release mechanism comprising a pressure release valve capable of releasing the gas to the atmosphere; and
a control unit that adjusts the initial position of the diaphragm by controlling the reciprocating pump and the pressure release mechanism,
wherein the control unit advances or retracts the plunger to a predetermined pressure application position, closes the pressure release valve after opening the pressure release valve and releasing the gas to the atmosphere, and then moves the plunger to an adjusted position which is a position of the plunger at which the diaphragm is located at a desired position.

6. The pressure sensing device according to claim 5, comprising:
a diaphragm position fixing mechanism capable of fixing the position of the diaphragm by closing the liquid flow path,
wherein the control unit opens the pressure release valve after the position of the diaphragm is fixed by the diaphragm position fixing mechanism.

7. The pressure sensing device according to any one of claims 1 to 6, comprising:
a socket part to which the pressure sensor is connected,
wherein the case and the diaphragm are provided so as to be removable from the socket part.

8. A blood purification apparatus, comprising:
the pressure sensing device according to any one of claims 1 to 7 as at least one of pressure sensing devices provided on a blood circuit to extracorporeally circulate blood of a patient, a liquid supply flow path to supply a supply liquid to the blood circuit or to a blood purifier provided on the blood circuit, and a waste liquid flow path to discharge a waste liquid from the blood purifier.
